# EUROPEAN PATENT APPLICATION

(11) **EP 2 505 151 A1**
(43) Date of publication of application: **03.10.2012**
(21) Application number: 10833299.0
(22) Date of filing: 26.11.2010
(51) Int. Cl.: A61B 17/3211

(54) **MEDICAL KNIFE**

(30) Priority: 26.11.2009 JP 2009268787
(71) Applicant: ACP Japan Co. Ltd., Tokyo 113-0033 (JP); Nakamura, Shoichi, Higashichikuma-gun Nagano 399-7502 (JP)
(72) Inventor: NAKAMURA, Shoichi, Higashichikuma-gun Nagano 399-7502 (JP)
(74) Representative: Gassner, Wolfgang
(86) International application number: PCT/JP2010/071076
(87) International publication number: WO 2011/065453

(57) **Abstract**

To provide a medical knife which enables the angle of the blade with respect to the grasp portion to be varied with ease without requiring attaching and detaching of the blade and is safe, hygienic and excellent in visibility, the medical knife has a knife body having a grasp portion, a blade and a blade holder portion for holding the blade, a blade cover that covers part of the knife body, and a lock portion that permits the blade cover to be locked in a storage portion for storing the blade in the knife body and in an exposure position for exposing the blade from the knife body, the lock portion permits the blade cover and the knife body to be locked in the storage position and a plurality of exposure positions, and the angles of the blade exposed in the plurality of exposure positions are made mutually different.

## Description

### Technical Field

The present invention relates to a knife such as a surgical knife used in the medical field, and more particularly, to a medical knife enabling an angle of the blade to be adjusted.

### Background Art

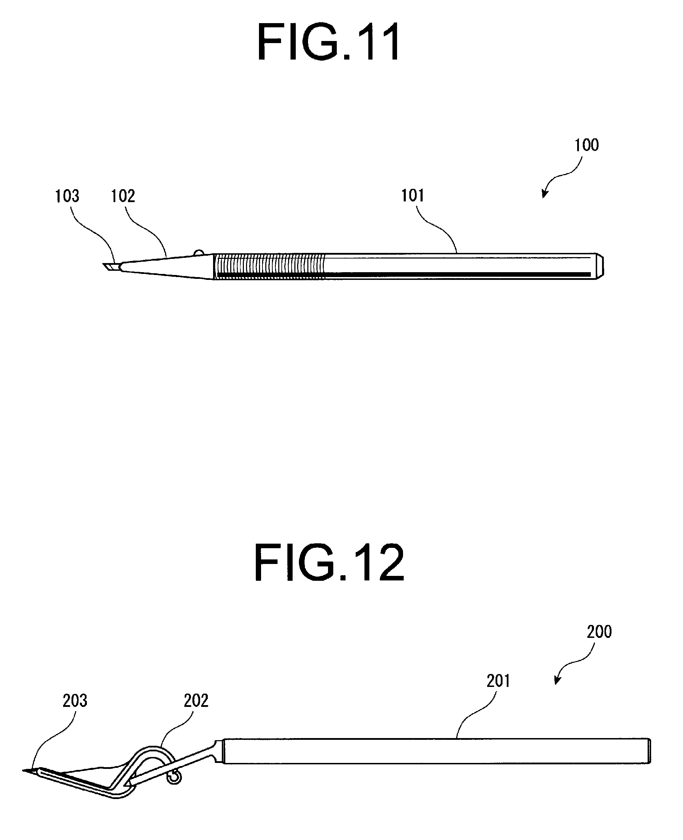

Conventionally, in a medical knife, as shown in FIG. 11, it is general that a grasp portion and a blade are positioned substantially in the straight line. In a conventional medical knife 100 shown in FIG. 11 as an example, a grasp portion 101, a blade holder 102 provided at the end portion of the grasp portion 101, and a blade 103 positioned at the tip of the blade holder 102 are arranged substantially in the straight line fixedly.

However, for example, in heart coronary-artery bypass surgery, from the relationship between the position of a blood vessel to cut and an insertion angle of the medical knife, the conventional medical knife as shown in FIG. 11 sometimes hides the knife front end in the hand of the operator, and has the problem of being hard to see and use in cutting. In other words, in the conventional medical knife with the pointed blade at the tip, as shown in FIG, 11, it is general that the ridge side of the blade is substantially parallel to the grasp portion, and that the blade side of the blade is angled with respect to the grasp portion. Such a conventional medical knife is usually used with the blade side of the blade pointed downward. At this point, since the cutting edge of the blade is positioned above the center line of the grasp portion, the cut area is easy to come within sight from the top. However, as in the above-mentioned heart surgery, when it is necessary to use the knife with the blade side of the blade pointed upward corresponding to the position of the affected area, the shape of the conventional medical knife provides seriously poor visibility of the cut area.

Therefore, such medical knives have previously been commercially available that a detachable blade can be disposed at an arbitrary angle with respect to the grasp portion (for example, see Patent Document 1) . FIG. 12 shows the medical knife described in Patent Document 1. The medical knife 200 is comprised of a grasp portion 201, and a blade holder 202 provided with a blade 203 at the tip, while being attachable at an arbitrary angle with respect to the grasp portion 201. Therefore, it is possible to ensure good visibility by varying the angle of the blade 203 with respect to the grasp portion 201 corresponding to the position of the affected area and/or preferences of the operator.

### Citation List

### Patent Literature

Patent Document 1: Japanese Patent Gazette No. 3980989

### Summary of Invention

### Problems to be Solved by the Invention

However, the conventional medical knife 200 as shown in FIG. 12 described above requires work of attaching the blade holder 202 to the grasp portion 201 in using. Further, also when the operator wants to vary the angle of the blade 203, it is necessary to conduct work of re-attaching the blade holder 202.

There is a high possibility that the hand, finger and the like of the operator may be wounded in such work of attaching and detaching the blade holder having the sharp blade to/from the grasp portion. Further, there are a possibility that the operator contacts the blade required to be always kept clean before using and causes saprophytic bacteria and the like to adhere, and a risk that the operator contacts the used blade with bacteria and the like adhering thereto. These are significantly serious problems in the medical institution for treating infectious diseases, etc.

Therefore, in view of the conventional circumstances as described above, it is an object of invention to provide a medical knife which enables the angle of the blade with respect to the grasp portion to be varied with ease without requiring attaching and detaching of the blade and is capable of being used in any position, while being safe, hygienic and excellent in visibility (viewability of the knife operating portion).

### Means for Solving the Problem

To attain the above-mentioned object, the invention provides a medical knife which is provided with a knife body having a grasp portion, a blade and a blade holder portion for holding the blade, a blade cover that covers part of the knife body, and a lock portion that permits the blade cover to be locked in a storage position for storing the blade in the knife body and in an exposure position for exposing the blade from the knife body, and which is characterized in that the blade holder portion is provided with a movable portion that has a first engagement portion and a second engagement portion for engaging in the blade cover while holding the blade, and an elastic support portion for elastically supporting the movable portion rotatably in a direction perpendicular to the axis, the blade cover is provided with a first guide portion and a second guide portion for engaging in the first engagement portion and the second engagement portion, the first engagement portion has a first slide-contact surface for engaging in the first guide portion to guide the movable portion in the axis direction, while coming into slide-contact with a corner portion of the first guide portion, and rotates the movable portion on the contact as the center, the second engagement portion has a second slide-contact surface comprised of a plurality of planes with different angles for engaging in the second guide portion to guide the movable portion in the axis direction, while stepwise coming into slide-contact with a guide surface of the second guide portion, and thereby guides rotation of the movable portion, the lock portion permits the blade cover and the knife body to be locked in the storage position and a plurality of exposure positions, and that the blade exposed in the plurality of exposure positions is inclined different angles respectively according to inclined angles of the second slide-contact surface.

Herein, it is suitable that the blade side of the blade is disposed on the side on which the blade is bent.

Further, it is preferable that a notch portion is provided at the end portion on the exposure side of the blade of the blade cover.

Furthermore, it is suitable that the cross-sectional shape of an outer region of the blade cover is the shape of an ellipse or a capsule.

### Advantageous Effects of Invention

According to the medical knife of the invention, it is not necessary to attach and detach the blade, and it is possible to set the angle of the blade with respect to the grasp portion at any angle among a plurality of beforehand set angles by one-touch operation. Further, it is possible to vary the angle by one-touch operation without contacting the blade, and to provide the medical knife which is safe, hygienic and excellent in visibility.

### Brief Description of Drawings

FIG. 1 contains entire schematic views of a medical knife according to an Embodiment of the invention;
FIG. 2 contains schematic views of a knife body in FIG. 1;
FIG. 3 contains schematic views of a blade cover in FIG. 1;
FIG. 4 contains schematic views to explain each stage of lock release operation and lock operation of the medical knife as shown in FIG. 1;
FIG. 5 contains schematic views to explain action in the lock release operation and lock operation of the knife body and blade cover of the medical knife as shown in FIG. 1;
FIG. 6 is a schematic view illustrating a movable portion of the medial knife as shown in FIG. 1;
FIG. 7 is a schematic view illustrating a state in which the movable portion strikes an end portion (corner portion) of a guide hole in the medical knife as shown in FIG. 1;
FIG. 8 contains schematic views (part 1) of the medical knife of FIG. 1 in a lock state in each lock position;
FIG. 9 contains schematic views (part 2) of the medical knife of FIG. 1 in a lock state in each lock position;
FIG. 10 is a schematic view showing an example in which a round blade with a round shape at the tip is applied to the medical knife according to an Embodiment of the invention;
FIG. 11 is a schematic view (part 1) showing an example of an appearance of a conventional medical knife; and
FIG. 12 is a schematic view (part 2) showing an example of an appearance of a conventional medical knife.

### Description of Embodiments

An Embodiment of the invention will specifically be described below with reference to drawings.

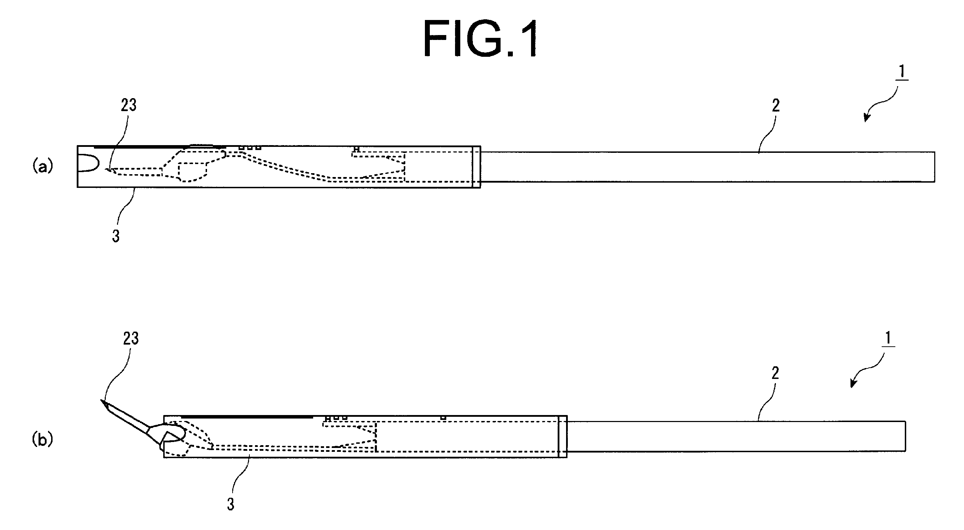

FIG. 1 contains entire schematic views of a medical knife according to an Embodiment of the invention. FIG. 1(a) shows a state in which a blade is stored inside a blade cover, and FIG. 1(b) shows a state in which the blade is exposed from the blade cover. As shown in the figures, a medical knife 1 of the invention is comprised of a knife body 2 and blade cover 3.

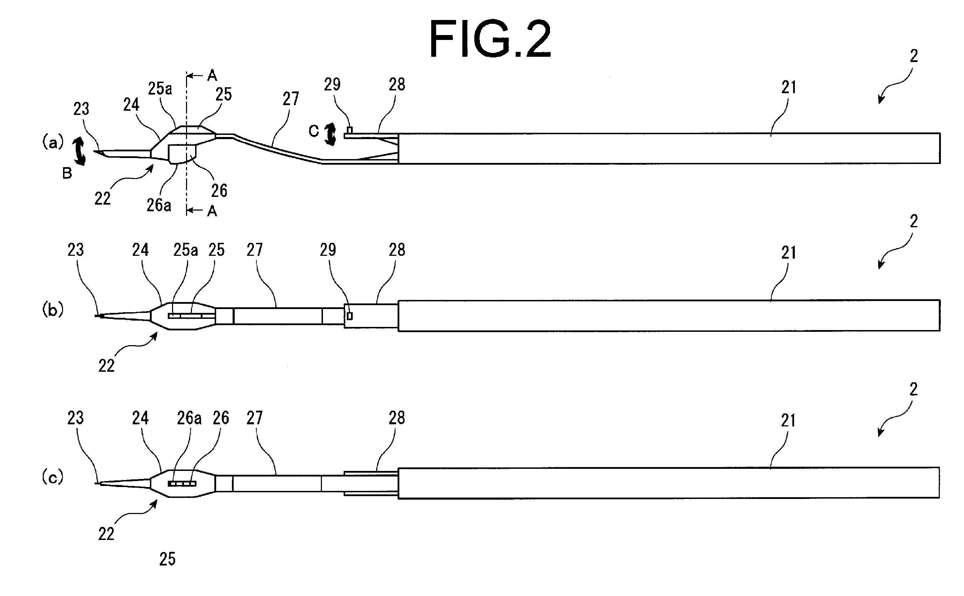

The knife body 2 will be described first. FIG. 2 shows a schematic configuration of the knife body. FIG. 2(a) is a front view of the knife body, FIG. 2(b) is a top view of the knife body, and FIG. 2 (c) is a bottom view of the knife body. Further, FIG. 2(d) is a sectional view [sectional view taken along line A-A in FIG. 2 (a)] of a movable portion. As shown in these figures, the knife body 2 is comprised of a rod-shaped grasp portion 21, a blade holder portion 22 for holding a blade 23, and a bridge portion 28. In addition, it is suitable that the blade holder portion 22, grasp portion 21 and bridge portion 28 except the blade 23 are integrally formed using resin.

The grasp portion 21 is a portion of the haft grasped by an operator in using the medical knife 1, and has a moderately thin long shape. A concavo-convex shape or the like may be formed on the surface as antiskid structure.

The blade holder portion 22 is provided with the blade 23, movable portion 24 for holding the blade 23, and a plate spring portion (elastic support portion) 27 for coupling the movable portion 24 and the grasp portion 21.

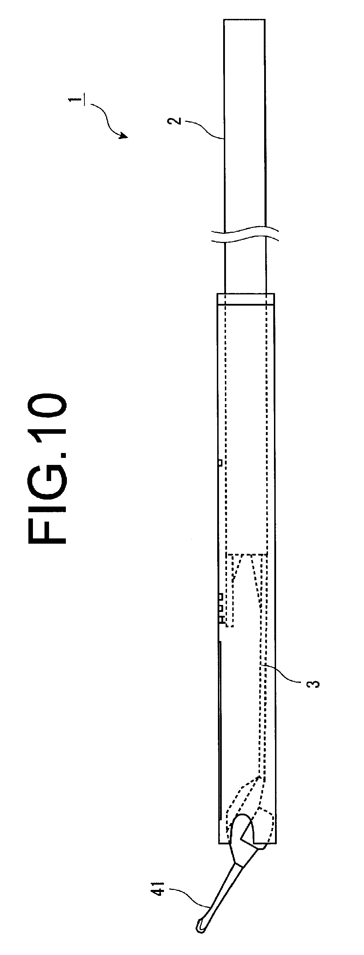

The blade 23 may be of material such as stainless steel for use in normal medical knives. Herein, a sharp blade with a sharp-pointed shape at the tip is shown in the figure as an example, and as shown in FIG. 10, a round blade 41 with a round shape at the tip may be applied to the medical knife 1 of the invention.

Further, herein, such an example is shown in the figure that the blade 23 is attached so that the blade side of the blade 23 points toward the backbone side (which is the upper side in normal use) of the medical knife 1. In other words, the blade side of the blade 23 points toward the bent side of the knife body 2. Such a configuration is an optimal configuration when the operator wants to point the blade side of the blade upward to use as in the heart surgery as described previously. As in the usual way, it is certainly possible to adopt a configuration with the blade of the blade 23 pointed toward the belly side of the medical knife 1. Also in this case, since the knife body 2 is bent toward the backbone side, it is possible to obtain the medical knife 1 excellent in visibility.

The movable portion 24 is a member for holding the blade 23 at the front end thereof, while shifting the blade 23 in the arrow B direction of FIG. 2 (a) by operating the knife body 2 and blade cover 3 as described later. In the movable portion 24, a first protrusion portion (first engagement portion) 25 is formed on the side (upper side in FIG. 2) to shift in exposing the blade 23 from the blade cover 3, while a second protrusion portion (second engagement portion) 26 is formed on the side (lower side in FIG. 2) to shift in storing the blade 23 in the blade cover. These protrusion portions 25, 26 engage respectively in a guide hole (first guide portion) 31 and guide groove (second guide portion) 32 of the blade cover 3 described later to guide the movable portion 24 to a predetermined position while regulating the movable area of the movable portion 24.

The plate spring portion 27 couples the grasp portion 21 and the movable portion 24, bends as shown in FIG. 2 (a) in an ordinary state, and when the blade 23 is exposed from the blade cover 3 and the movable portion 24 is shifted, expands according thereto. Further, when the plate spring portion 27 is once locked, the portion 27 recovers by elasticity and maintains the lock state, while permitting rotation in the circumference direction added in locking a protrusion portion (lock portion) 29 provided in the bridge portion 28 in a lock groove 34 of an engagement hole 33, and in releasing the lock.

The bridge portion 28 is a member extending in the shape of a cantilever from the grasp portion 21, and is capable of shifting in the arrow C direction of FIG. 2(a) from a connection part with the grasp portion 21 as a base point so as to enable the blade cover 3 to be attached in manufacturing. The bridge portion 28 is provided with the protrusion portion (lock portion) 29 for engaging in the engagement hole 33 of the blade cover 3. By locking the protrusion portion 29 in the lock groove 34 of the engagement hole 33, it is possible to hold the knife body 2 in the exposure position or storage position with respect to the blade cover 3.

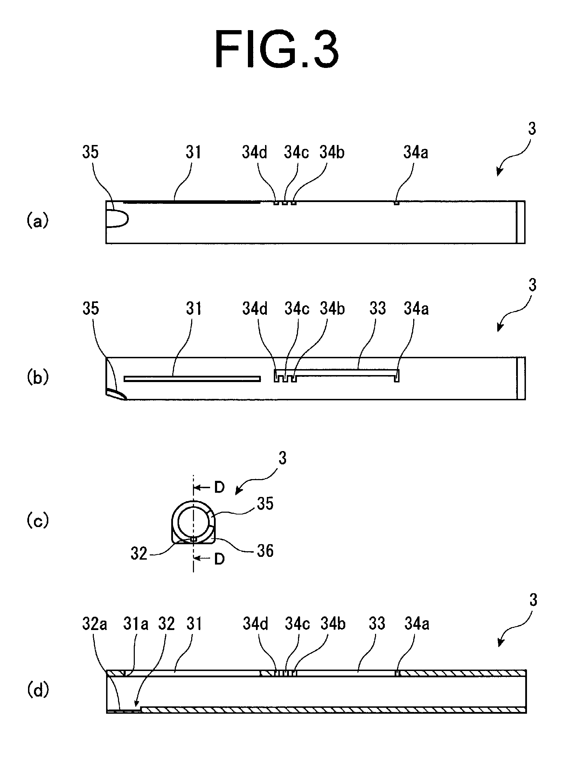

The blade cover 3 will be described later. FIG. 3 shows schematic diagrams of the blade cover. FIG. 3(a) is a front view of the blade cover, FIG. 3(b) is a top view of the blade cover, FIG. 3 (c) is a left side elevational view of the knife body, and FIG. 3(d) is a sectional view [sectional view taken along line D-D in FIG. 3(c)] of the blade cover. As shown in these figures, the blade cover 3 is formed in a substantially cylindrical shape, and is provided with the guide hole (first guide portion) 31 for engaging in the first protrusion portion 25 of the movable portion 24 near the end portion on the exposure side of the blade 23. On the inside surface in the position opposed to the guide hole 31, the guide groove (second guide portion) 32 is formed to engage in the second protrusion portion 26 of the movable portion 24.

The blade cover 3 further has the engagement hole 33 in the position closer to the grasp portion 21 side than the guide hole 31. In the engagement hole 33 is engaged the protrusion portion 29 provided in the bridge portion 28. Then, a plurality of (at least three or more) lock grooves 34 is formed in the engagement hole 33, and by locking the protrusion portion 29 in the lock groove 34 in any given position, it is possible to set the blade 23 for a desired angle. Herein, shown is the case having four lock grooves, 34a, 34b, 34c, 34d.

It is suitable that the blade cover 3 is further provided with a notch portion 35 at part of the front end. By thus notching part of the front end of the blade cover 3, it is possible to remove an area obstructing the view of the blade 23 of the medical knife 1, and to improve visibility. It is desirable that the notch portion 35 is provided in an obliquely upward position as shown in FIG. 3(a). Herein, it is noted that the vicinity of the center of the backbone side (which is the upper side in normal use) of the blade cover 3 is nearby the trajectory such that the blade 23 passes through in the operation of exposing or storing the blade 23. When the notch portion 35 is provided in this position, the risk is high, and therefore, to avoid the risk, the notch portion 35 is suitably disposed in an obliquely upward position.

The notch portion 35 may be provided on both left and right sides, or may be formed on either the left or right side with consideration given to the dominant hand of the operator. Herein, shown in the figure is the medical knife 1 optimal for use by right-handed operators in which the notch portion 35 is positioned on the left side assuming that the grasp portion 21 is at the front, the blade is at the back, and that the backbone side of the blade cover 3 is up.

In addition, the configuration with the notch portion 35 formed in the blade cover 3 is described above, but the invention is not limited thereto to improve visibility, and various devices are effective such that a taper is provided on the outer surface at the front end portion on the blade exposure side of the blade cover 3, and that only part is cut, or the like.

In addition, the blade cover 3 as shown in FIG. 3 shows an example in which a stopper 36 to prevent rolling is provided in part of the belly side (which is the lower side in normal use) of the blade cover 3.

Described next is the action in blade exposure operation and blade storage operation in the medical knife configured as described above.

In the following description, such an example will specifically be descried that the blade 23 can be inclined three angles [with respect to the reference (storage time), for example, substantially 0 degree (horizontal state), 20 degrees and 30 degrees] and fixed in exposing the knife body 2 from the blade cover 3. In the following description, it is assumed that a position for locking the knife body 2 in a storage state is a storage position L1, a position for exposing the knife body 2 from the blade cover 3 and locking the knife body 2 at an inclination of 0 degree from the reference is a lock position L2, a position for locking the knife body 2 at an inclination of 20 degrees is a lock position L3, and that a position for locking the knife body 2 at an inclination of 30 degrees is a lock position L4.

As shown in FIG. 3, the engagement hole 33 formed in the blade cover 3 is provided with a plurality of (herein, four) lock grooves 34a, 34b, 34c, 34d. Among these lock grooves, the lock groove 34a to lock the knife body 2 in the storage position L1 is in the position nearest the grasp portion 21 side of the knife body 2. Then, toward the exposure direction of the knife body 2, arranged are the lock groove 34b to lock the knife body 2 in the lock position L2, the lock groove 34c to lock the knife body 2 in the lock position L3, and the lock groove 34d to lock the knife body 2 in the lock position L4, in this order.

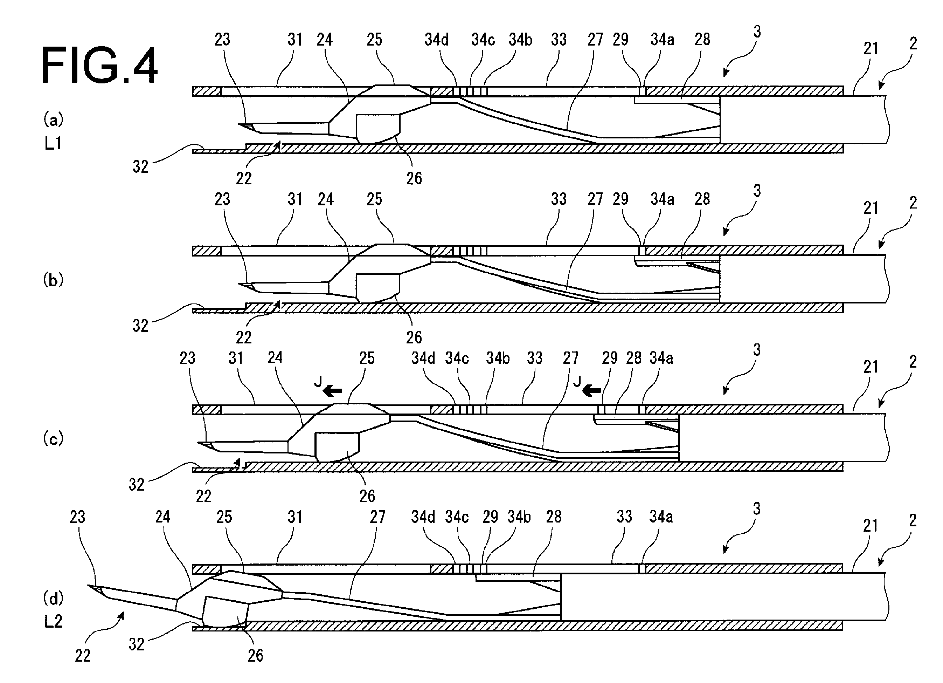

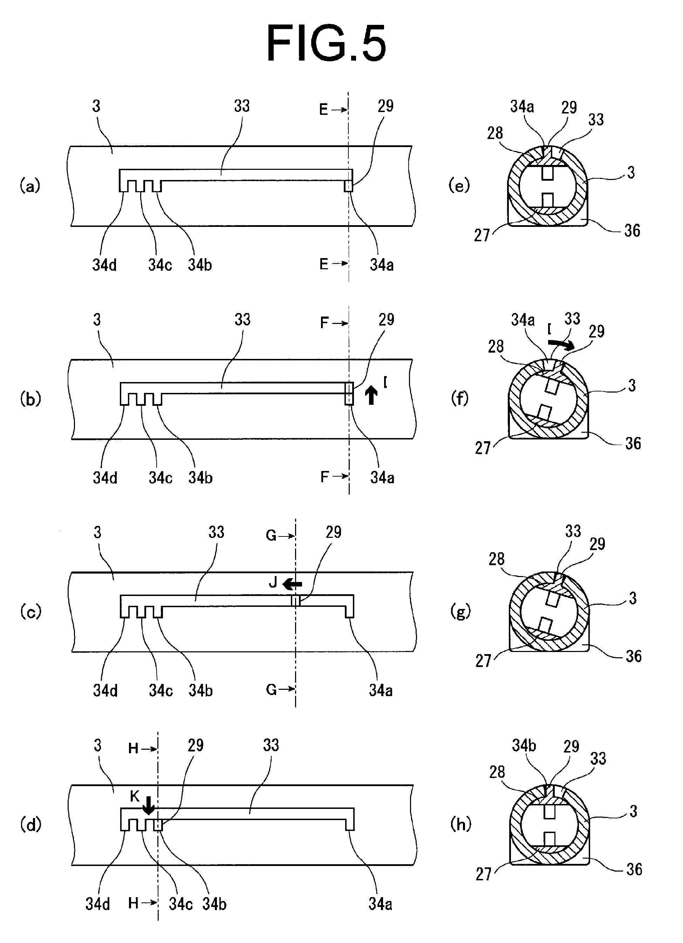

Described first are the lock release operation and lock operation in the knife body 2 and blade cover 3. Described herein is the case of operating the knife body 2 in a lock state in the storage position L1 and locking the knife body 2 in the lock position L2 again. FIGs. 4 and 5 are schematic views to explain the action in the lock release operation and lock operation of the knife body 2 and blade cover 3. FIGs. 4 (a) to 4 (d) are schematic views of the medical knife 1 in each stage in the lock release operation and lock operation, FIGs. 5 (a) to 5 (d) are partial enlarged top views of the periphery of the engagement hole 33, FIG. 5(e) is a sectional view taken along E-E line in FIG. 5(a), FIG. 5(f) is a sectional view taken along F-F line in FIG. 5(b), FIG. 5 (g) is a sectional view taken along G-G line in FIG. 5(c), and FIG. 5(h) is a sectional view taken along H-H line in FIG. 5(d).

FIGs. 4 (a), 5 (a) and 5 (e) show the state in which the knife body 2 is locked in the storage position L1. At this point, the protrusion portion 29 of the bridge portion 28 is locked in the lock groove 34 and stabilized.

To release this lock state, while grasping the grasp portion 21 of the medical knife 1 and holding the blade cover 3, the grasp portion 21 is rotated in the arrow I direction in FIGs. 5 (b) and 5 (f) with respect to the blade cover 3. Then, in response thereto, the bridge portion 28 and the base portion of the plate spring portion 27 are also rotated in the arrow I direction. However, at this point, as shown in FIG. 4(b), since the first protrusion portion 25 of the movable portion 24 is engaged in the guide hole 31, the rotation of the entire blade holder 22 is prohibited. As a result, as shown in FIG. 4(b), the plate spring portion 27 is twisted. By this means, in the blade holder 22, the movable portion 24 does not rotate, and only the base portion side of the plate spring portion 27 rotates. Meanwhile, the entire bridge portion 28 rotates, following the rotation of the grasp portion 21. By this rotation, as shown in FIGs. 5 (b) and 5 (f), the protrusion portion 29 withdraws from the lock groove 34a, and the lock in the lock position L1 is released.

Next, to lock the knife body 2 in the lock position L2, first, the knife body 2 is shifted to slide in the arrow J direction as shown in FIGs. 4(c) and 5(c) with respect to the blade cover 3. At this point, since the protrusion portion 29 is inhibited from shifting in the circumference direction by the engagement hole 33, the bridge portion 28 and the base portion of the plate spring portion 27 shift while maintaining the twist shown in FIG. 4(b).

When the protrusion portion 29 is slid to the position of the lock groove 34b, as shown in FIGs. 4(d), 5(d) and 5(h), the portion 29 is engaged in the lock groove 34b [the arrow K direction of FIG. 5(d)]. The twisted plate spring portion 27 elastically recovers, and the protrusion portion 29 is engaged inside the lock groove 34b with stability unless the force of rotating the knife body 2 is applied again.

The above-mentioned lock release operation and lock operation are almost the same in the case of performing on any lock groove. In addition, in the foregoing, in withdrawing the protrusion portion 29 from the lock groove 34a that locks in the storage position L1, it is described that the bridge portion 28 and only the base portion of the plate spring portion 27 are rotated in rotating the grasp portion 21 because the first protrusion portion 25 of the movable portion 24 is engaged in the guide hole 31 of the blade cover 3. In contrast thereto, in the cases of withdrawing the protrusion portion 29 from the lock grooves 34b, 34c, 34d respectively locked in the lock positions L2, L3, L4 that expose the blade 23, the first protrusion portion 25 is withdrawn or almost withdrawn from the guide hole 31. However, instead thereof, the second protrusion portion 26 of the movable portion 24 is engaged in the guide groove 32 of the blade cover 3. Therefore, as a result, as in the same way described above, in response to the rotation of the grasp portion 21, the bridge portion 28 and only the base portion of the plate spring portion 27 rotate.

Described next is a configuration for making the angle of the movable portion 24 (blade 23) variable and the action thereof.

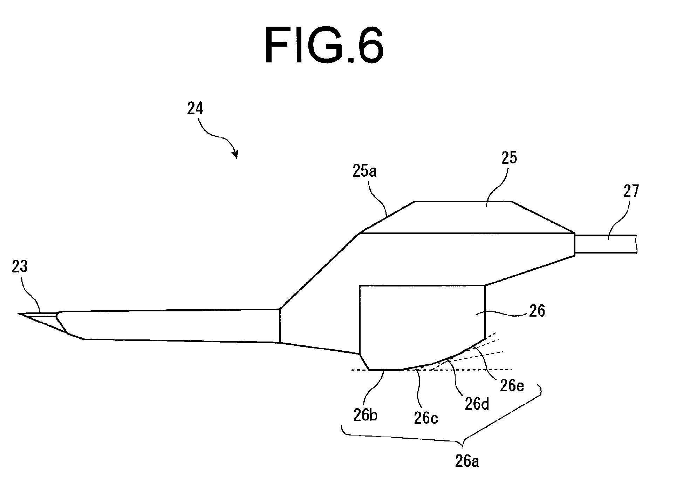

First, FIG. 6 shows the enlarged movable portion 24. As shown in the figure, the second protrusion section 26 is provided with a slide-contact surface (second slide-contact surface) 26a coming into contact with the inner surface of the blade cover 3 or the bottom 32a of the guide groove 32. Herein, the slide-contact surface 26a is comprised of slide-contact surfaces 26b, 26c, 26d, 26e for forming four angles.

The slide-contact surface 26b is a surface for coming into contact with the inner surface of the blade cover 3 or the bottom 32a of the guide groove 32 when the knife body shifts to the lock position L2 from the storage position L1. Next, the slide-contact surface 26c is a surface for coming into contact with the bottom 32a of the guide groove 32 when the knife body 2 is in the lock position L2. The slide-contact surface 26c has a predetermined angle θ₁ (herein, 10 degrees) with respect to the slide-contact surface 26b that is the reference (in storage) .

Then, the slide-contact surface 26d is a surface for coming into contact with the bottom 32a of the guide groove 32 when the knife body 2 is in the lock position L3. The slide-contact surface 26d has a predetermined angle θ₂ (herein, 20 degrees) with respect to the slide-contact surface 26b that is the reference (in storage). Further, the slide-contact surface 26e is a surface for coming into contact with the bottom 32a of the guide groove 32 when the knife body 2 is in the lock position L4. The slide-contact surface 26c has a predetermined angle θ₃ (herein, 30 degrees) with respect to the slide-contact surface 26b that is the reference (in storage).

Thus, the second protrusion portion 26 is provided with the slide-contact surface 26b that is the reference and a plurality of slide-contact surfaces, 26c, 26d, 26e, having respective different angles (θ₁, θ₂, · · · θₙ; herein, θ₁ < θ₂< · · · < θₙ) with respect to the slide-contact surface 26b as the reference. By this means, it is possible to incline the movable portion 24 each arbitrary angle, θ₁, θ₂, · · · θₙ.

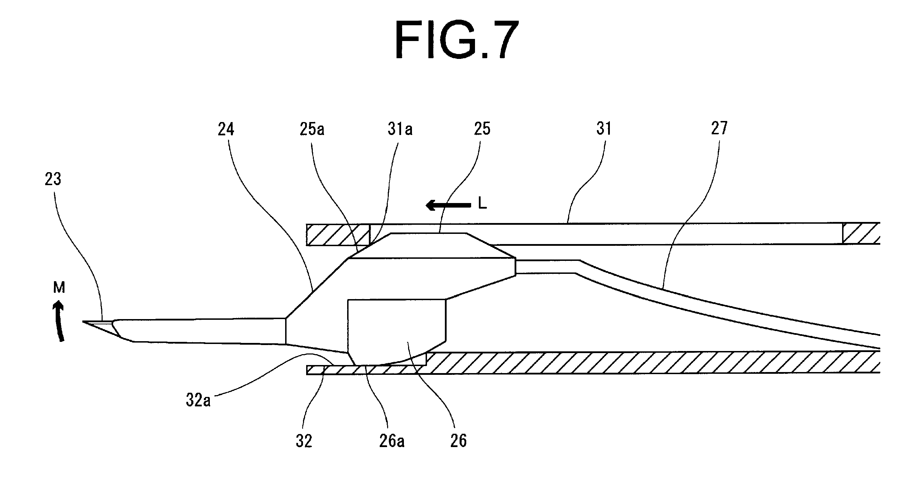

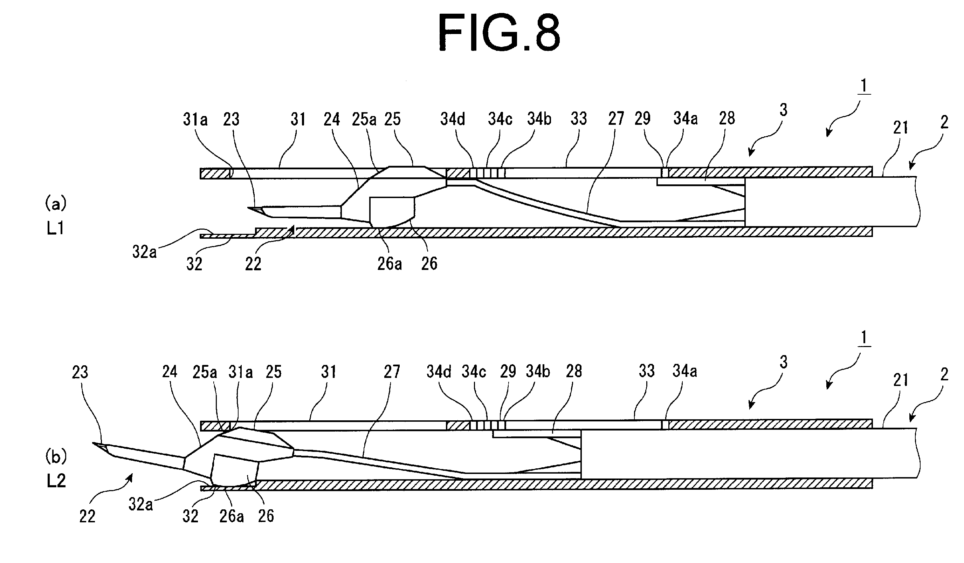

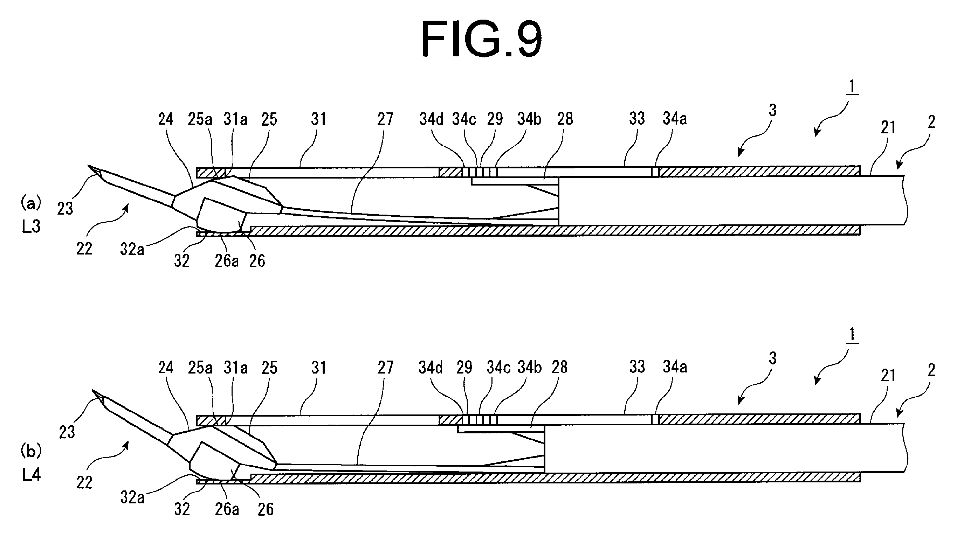

The actual action will specifically be described below. FIG. 7 is a schematic view illustrating a state in which the movable portion 24 strikes an end portion (corner portion) 31a of the guide hole 31. Further, FIGs. 8 and 9 are schematic views of the medical knife 1 in a lock state in each lock position. FIG. 8 (a) shows a state in which the knife 1 is locked in the storage position L1, FIG. 8(b) shows a state in which the knife 1 is locked in the lock position L2, FIG. 9 (a) shows a state in which the knife 1 is locked in the lock position L3, and FIG. 9(b) shows a state in which the knife 1 is locked in the lock position L4.

In FIG. 8(a), as described above, in the movable portion 24, the first protrusion portion 25 engages in the guide hold 31, while the slide-contact surface 26b of the second protrusion portion 26 is brought into contact with the inner surface of the blade cover 3. When the lock is released from this state and the knife body 2 is shifted with respect to the blade cover 3, as shown in FIG. 7, the blade 23 is exposed from the blade cover 3, and the second protrusion portion 26 fits and engages in the guide groove 32, while the first protrusion portion 25 strikes the end portion (corner portion) 31a of the guide hole 31. As shown in FIG. 6, the first protrusion portion 25 has the slide-contact surface (first slide-contact surface) 25a to strike the end portion 31a of the guide hole 31.

When the knife body 2 is further shifted with respect to the blade cover 3 in this state, while shifting in the arrow L direction of FIG. 7, the movable portion 24 strikes at the slide-contact surface 25a the end portion 31a of the guide hole 31 to slide, and rotates gradually in the arrow M direction of FIG. 7. According to the rotation, the bending of the plate spring portion 27 gradually extends. Further, in response to the rotation, also the second protrusion portion 26 rotates, while gradually sliding at the slide-contact surface 26b on the bottom 32a of the guide groove 32. Then, when the bottom 32a of the guide groove 32 comes into contact with the adjacent slide-contact surface 26c, the angle of the blade 23 is inclined θ₁ degrees (herein, 10 degrees) from the reference. The lock groove 34b is disposed in a position for enabling the protrusion portion 29 of the bridge portion 28 to be locked in this state. Therefore, when locking in this position (lock position L2), the movable portion 24 is fixed while being inclined θ₁ degrees from the reference, and it is possible to obtain the medical knife 1 with the blade 23 inclined θ₁ degrees with respect to the grasp portion 21.

In the same way as described above, the lock grooves 34c, 34d are disposed in positions for enabling the protrusion portion 29 of the bridge portion 28 to be locked in a state in which the bottom 32a of the guide hole 32 comes into contact with the slide-contact surface 26d or 26e and the angle of the blade 23 is inclined θ₂ degrees or θ₃ degrees from the reference, respectively. Accordingly, in the case of needing the medical knife 1 inclined θ₂ degrees or θ₃ degrees with respect to the grasp portion 21, the knife body 2 is further shifted in the exposure direction with respect to the blade cover 3, the movable portion 24 rotates and comes into contact with the bottom 32a of the guide groove 32 on the slide-contact surface 26d or 26e, and the protrusion portion 29 of the bridge portion 28 is thereby locked in the lock groove 34c or 34e (lock position L3 or L4) with the blade 23 inclined θ₂ degrees or θ₃ degrees from the reference, respectively.

In addition, when the lock is released from the above-mentioned lock position L2, L3 or L4 and the knife body 2 is returned to the original storage position L1, the knife body 2 is rotated to withdraw the protrusion portion 29 of the bridge portion 28 from the lock groove 34b, 34c or 34d, and the knife body 2 is further shifted in the storage direction. Then, the movable portion 24 backs while rotating in the direction opposite to the arrow M of FIG. 7, and the plate spring portion 27 returns and bends to the original state. Further, the first protrusion portion 25 engages again in the guide hole 31, and the second protrusion portion 26 withdraws from the guide groove 32. The knife body 2 is shifted backward inside the blade cover 3 without change, and the protrusion portion 29 of the bridge portion 28 is slid to the position of the lock groove 34a, and is locked in this position. Thus, the medical knife 1 is capable of being stored safely.

In addition, in the foregoing, the example is shown where the cross-sectional shapes of the grasp portion 21 of the knife body 2 and blade cover 3 are round, but may be elliptical, capsule type, polygon, combination thereof, and the like. In the invention, since the grasp portion 21 is rotated to lock or withdraw the protrusion portion 29 provided in the bridge portion 28 in/from the lock groove 34 of the engage hole 33, it is necessary that the grasp portion 21, bridge portion 28 and the base portion of the plate spring portion 27 have the shape capable of rotating inside the blade cover 3, and as long as the rotation is allowed, any shape is applied. Particularly, in the case where the shape of an ellipse or a capsule is applied to the cross-sectional shape of the outer region of the blade cover, when the major-axis direction is set at the up/down direction, the portion obstructing the view is fewer than that in the circle of which the diameter is the same as the length of the major axis, and therefore, such a shape is excellent in visibility.

In addition, in the foregoing, the installation angle of the blade 23 is beforehand bent with respect to the grasp portion 21. By this means, it is possible to increase the bent angle in exposing the blade 23. As a matter of course, the blade 23 may be attached in parallel with the grasp portion 21.

By configuring as described above, according to the medical knife of the invention, it is possible to set the angle of the blade with respect to the grasp portion at any angle among a plurality of beforehand set angles by one-touch operation without requiring attaching and detaching of the blade. Further, the angle is capable of being varied by one-touch operation with no need for contacting the blade, and it is possible to provide the medical knife that is safe, hygienic and excellent in visibility.

The Embodiment of the invention is described as mentioned above, but the invention is not limited to the above-mentioned Embodiment, is capable of being modified in various manners based on the subject matter of the invention, and is intended not to exclude the modifications from the scope of the invention.

### Industrial Applicability

The invention relates to a knife such as a surgical knife used in the medical field, more particularly to a medical knife enabling an angle of the blade to be adjusted, and has industrial applicability.

### Reference Signs List

- 1: Medical knife
- 2: Knife body
- 3: Blade cover
- 21: Grasp portion
- 22: Blade holder portion
- 23: Blade
- 24: Movable portion
- 25: First protrusion portion (first engagement portion)
- 25a: Slide-contact surface (first slide-contact surface)
- 26: Second protrusion portion (second engagement portion)
- 26a: Slide-contact surface (second slide-contact surface)
- 26b, 26c, 26d, 26e: Slide-contact surface
- 27: Plate spring portion (elastic support portion)
- 28: Bridge portion
- 29: Protrusion portion (lock portion)
- 31: Guide hole (first guide portion)
- 31a: End portion (corner portion) of the guide hole
- 32: Guide groove (second guide portion)
- 32a: Bottom of the guide groove
- 33: Engagement hole
- 34, 34a, 34b, 34c, 34d: Lock groove
- 35: Notch portion
- 36: Stopper
- 41: Round blade

## Claims

1. A medical knife comprising:
a knife body having a grasp portion, a blade and a blade holder portion for holding the blade;
a blade cover that covers part of the knife body; and
a lock portion that permits the blade cover to be locked in a storage position for storing the blade in the knife body and in an exposure position for exposing the blade from the knife body,
wherein the blade holder portion is provided with a movable portion that has a first engagement portion and a second engagement portion for engaging in the blade cover while holding the blade, and an elastic support portion for elastically supporting the movable portion rotatably in a direction perpendicular to the axis,
the blade cover is provided with a first guide portion and a second guide portion for engaging in the first engagement portion and the second engagement portion,
the first engagement portion has a first slide-contact surface for engaging in the first guide portion to guide the movable portion in the axis direction, while coming into slide-contact with a corner portion of the first guide portion, and rotates the movable portion on the contact as the center,
the second engagement portion has a second slide-contact surface comprised of a plurality of planes with different angles for engaging in the second guide portion to guide the movable portion in the axis direction, while stepwise coming into slide-contact with a guide surface of the second guide portion, and thereby guides rotation of the movable portion,
the lock portion permits the blade cover and the knife body to be locked in the storage position and a plurality of exposure positions, and the blade exposed in the plurality of exposure positions is inclined different angles respectively according to inclined angles of the second slide-contact surface.

2. The medial knife according to claim 1, wherein the blade side of the blade is disposed on the side on which the blade is bent.

3. The medical knife according to claim 1, wherein a notch portion is provided at the end portion on the exposure side of the blade of the blade cover.

4. The medical knife according to claim 1, wherein a cross-sectional shape of an outer region of the blade cover forms the shape of an ellipse or a capsule.
